# EUROPEAN PATENT APPLICATION

(11) **EP 0 674 874 A2**
(43) Date of publication of application: **04.10.1995**
(21) Application number: 95850032.4
(22) Date of filing: 10.02.1995
(51) Int. Cl.: A61B 5/12

(54) **Method and device for testing the reflexes of the stapedius**

(30) Priority: 14.02.1994 SE 9400474
(71) Applicant: ENTOMED AB, S-211 18 Malmö (SE)
(72) Inventor: Jönsson, Karl Erik, S-224 71 Lund (SE)
(74) Representative: Perklev, Karin Cecilia

(57) **Abstract**

In a method for testing the reflex of the stapedius, stimulus tone of varying intensity is generated, and one ear of the test subject is subjected to it. The reaction of the stapedius to this stimulus tone is probed.

When the intensity of the stimulus tone exceeds the stimulus threshold of the stapedius reflex, the latter is triggered, which has measurable effects on e.g. the eardrum.

By varying the intensity of the stimulus tone in such a manner that the degree of contraction of the stapedius is essentially maintained or increased, the test will take much less time than has hitherto been possible.

The stimulus tone can be continuous, in which case its intensity is varied according to an increasing function, or be a pulse signal, in which case the interval between the pulses is so brief that the stapedius has no time to relax between the pulses.

A device for implementing the method is also disclosed.

## Description

This invention relates to a method for testing the reflex of the stapedius, comprising the steps of generating a stimulus tone of variable intensity and subjecting at least one ear of the test subject to this tone; and probing the reaction of the stapedius to the stimulus tone. The invention further concerns a device for implementing the method.

In a clinical examination of the ear, different quantitative probing methods are used for characterising different components of the ear function. Thus, the testing of the stapedius reflex and its stimulus threshold is important tool when utilising audiological differential diagnostic techniques, investigating a case of facial paralysis, or suspecting a psychogenic hearing loss. The stapedius reflex is a reflex contraction of the stapedius, which is triggered by sound of a certain intensity and therefore is referred to as the acoustic stapedius reflex (ASR).

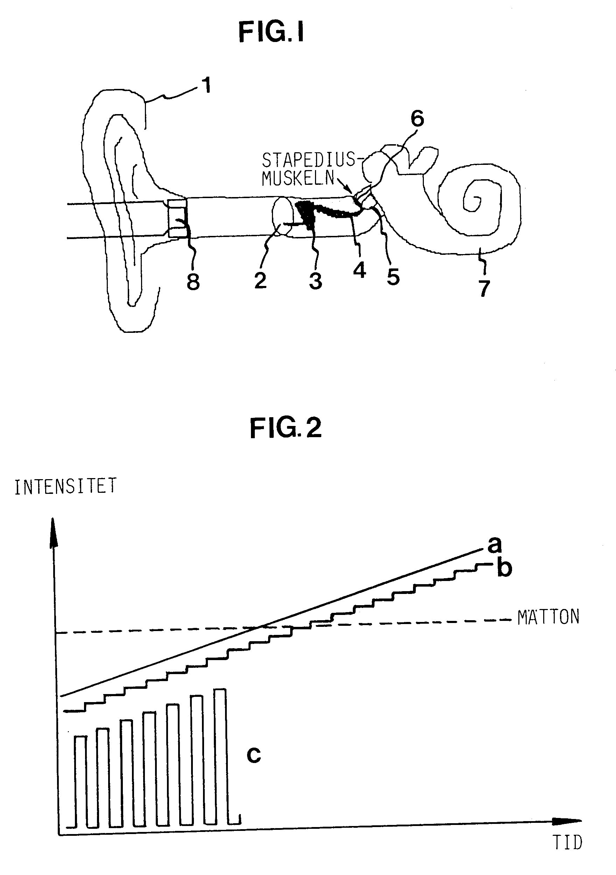

Fig. 1 schematically illustrates the anatomy of the ear and the middle ear. Thus, Fig. 1 shows the ear 1, the eardrum 2, the malleus 3, the incus 4, the stapes 5, the stapedius 6 and the liquid-filled cochlea 7. There is also shown a probe 8, which is inserted in the ear and is used for testing the stapedius reflex.

The stapedius 6, which is a small muscle in the middle ear, has one end attached to the stapes 5 (the stapedius bone) and another end attached to the wall of the middle ear. When the stapedius contracts, the stapes 5 is angled, which normally reduces the flexibility of the auditory ossicles 3, 4 and 5, and hence that of the eardrum 2. The middle ear will then react to sound to a lesser extent than would have been the case if the stapedius had not been contracted. From a physiological point of view, the stapedius reflex may be seen as a means for protecting the delicate mechanism of the inner ear against harmful sound levels.

In a healthy person, the contraction of the stapedius is triggered by loud sound, i.e. sound whose intensity exceeds a certain value, i.e. the stimulus threshold. In an unhealthy person, on the other hand, the ASR may be completely extinct or have a delayed or raised stimulus threshold. In an ASR test, an intact ASR is established and the sound intensity of the stimulus threshold is measured.

Today's ASR test involves a so-called impedance audiometer, which is provided with a probe 8 (see Fig. 1) which is so dimensioned that one end thereof can be inserted in the auditory meatus. The probe 8 is able to emit two different signals, of which one is referred to as the probe tone and the other is referred to as the stimulus tone. The probe tone may have a constant frequency as well as a constant intensity or be so varied that the sound pressure in the ear is maintained constant. As a rule, the intensity is in the range of 70-90 dB. By dB is here meant either dBSPL, i.e. dB Sound Pressure Level, or dBHL, i.e. dB Hearing Level. The choice of unit has no bearing on the implementation of the probing. The frequency and the intensity of the stimulus tone may be varied.

The probe 8 comprises a microphone, which registers the sound pressure generated by the probe tone in the auditory meatus and converts the sound to electric signals, which in turn can be analysed in the electronic part of the impedance audiometer.

The establishment of the ASR and its stimulus threshold is based on the measurement of the sound pressure in the auditory meatus. One of the effects of the stiffening of the eardrum caused by the contraction of the stapedius is that the acoustic impedance increases, resulting in a measurable increase of the sound pressure in the auditory meatus. By measuring the alteration of the sound pressure, one may obtain a measure of the alteration of the acoustic impedance and, consequently, of the degree of contraction of the stapedius. However, the measurement result is often given not as the impedance of the system but as its admittance, i.e. the inverted value of the impedance.

The probing is carried out as follows. The probe placed in the auditory meatus emits the stimulus tone and the probe tone and then registers the sound pressure generated by the probe tone. The probe tone is continuous, whereas the stimulus tone consists of a sequence of pulses, which each have a constant frequency but whose intensity is increased from one pulse to another, usually in steps of 5dB. In those instruments that are used today, each stimulus pulse lasts 1-2 sec and several seconds elapse between the pulses. When the intensity of the stimulus pulses passes the stimulus threshold of the stapedius reflex, the ASR is triggered, i.e. the stapedius contracts, which alters the sound pressure in the auditory meatus.

The impedance audiometer provides the measurement result in the form of a diagram showing the sound pressure as a function of the time, and/or in the form of a numerical value of the stimulus threshold, i.e. the lowest sound intensity that triggers the ASR.

At the beginning of the test, the stimulus pulse has an intensity well below the stimulus threshold. When the stimulus threshold is passed and the ASR is triggered, this will be visible in the diagram of the impedance audiometer. In order to establish the stimulus threshold with maximum accuracy, the test proceeds with an additional number of stimulus pulses of increasing intensity. Since there may occur qualitative alterations of the sound pressure registered other than an extinct ASR or a raised stimulus threshold, it is also useful to perform the test with sequences of stimulus pulses of different frequencies, mostly 500 Hz, 1000 Hz, 2000 Hz and 4000 Hz. As a rule, each sequence includes 5-10 pulses.

In today's impedance audiometers, the sound pressure generated by the probe tone is measured when the stimulus pulses are being emitted, when the stapedius is contracted if the ASR has been triggered, and for a second or so before and after the event, when the stapedius and the eardrum revert to the initial position. This technique is disadvantageous in that it requires a very steep filter for separating the probe tone from the stimulus tone.

As mentioned in the foregoing, the measurement involves a sequence of stimulus pulses of different intensities. The measurement of an entire sequence of stimulus pulses takes 0.5-1 min. Since the measurement is usually repeated with a number of different frequencies, it will take several minutes to carry it out.

In order that one should obtain reproducible results, it is essential that the position of the probe in the auditory meatus remains the same during the entire measurement, since every alteration of the measurement geometry produces a change of the impedance conditions. It sometimes happens that the entire measurement has to be repeated, as a result of displacements of the probe. Consequently, it is especially difficult to perform the measurement on patients having difficulty in cooperating.

As a result of the complexity of the test of the stapedius reflex, this important test method is utilised but to a limited extent.

One object of the invention is, therefore, to provide a method for testing the stapedius reflex, which is simpler and takes less time than the prior-art method.

Another object of the invention is to provide a device for implementing the inventive method.

These objects are achieved by a method and a device having the characteristics recited in appended claims 1 and 9, respectively.

The invention utilises the physiological phenomenon that the degree of contraction of the stapedius is proportional to the intensity of the stimulus tone when the stimulus threshold of the stapedius reflex has been passed. Thus, one does not have to give the stapedius time to relax between each stimulation in order to obtain an accurate test result, but the stapedius can, while still contracted, be subjected to another stimulation of increased intensity. As a result, the intensity of the stimulus tone can be changed much more often than before and may even be changed continuously, so that the test of the stapedius reflex can be performed in much less time. Furthermore, the intensity can be changed in smaller steps, enabling more accurate values of the stimulus threshold of the stapedius reflex.

In a first mode of implementation of the invention, the stimulus tone is continuously emitted and its intensity is varied according to an increasing function, for instance in small steps or continuously.

In a second mode of implementation, the stimulus tone is a pulse signal, in which the pulses are so frequent that the degree of contraction of the stapedius essentially does not have time to change between the pulses. The course of reaction of the stapedius is such that the reflex contraction occurs rapidly when there is a stimulation exceeding the stimulus threshold, but that the relaxation when the stimulation ceases is slow. Since the test involves a sequence of stimulus pulses of increasing intensity, the stapedius will normally exhibit a gradually increasing degree of contraction. Since the degree of contraction does not change between the pulses, the sound pressure can be measured between the stimulus pulses and there will no longer be any need of a filter for separating the probe tone and the stimulus tone.

In one preferred mode of implementation, there is less than 200 ms between the pulses. A complete test involving all the four frequencies mentioned above can be done in 12 sec, which is a vast improvement considering that a conventional test takes several minutes.

A mode of implementation of the invention will be described in more detail below with reference to the accompanying drawings, in which
Fig. 1, which has already been discussed, is a schematic view illustrating the anatomy of the ear and the middle ear and showing the position of the prcbe in the auditory meatus;
Fig. 2 is a diagram schematically illustrating different variants of how the intensity of the stimulus tone can be varied as a function of the time;
Fig. 3 is a block diagram illustrating the construction of an impedance audiometer according to the invention; and
Fig. 4 is a diagram showing the admittance of the middle-ear system as a function of the intensity of the stimulus tone.

Fig. 2 schematically illustrates how the intensity of the stimulus tone can be varied as a function of the time in different variants of the invention. The curves have been offset vertically in relation to one another to enable all the curves to be shown in one and the same diagram. Also, a probe tone is indicated by a dashed line. As appears from the diagram, the probe tone can be continuous and have a constant intensity. Also its frequency may be constant. As an example, it may be mentioned that the frequency of the probe tone may be 226 Hz and its sound pressure level be 90 dBSPL.

The intensity of the probe tone may also be varied, for instance in such a manner as to keep constant the sound pressure generated by the probe tone in the ear.

The curve a represents a variant in which the ear of the test subject is subjected to a continuous stimulus tone whose intensity increases according to a strictly increasing, continuous function.

The curve b represents a variant in which the stimulus tone is continuous but the intensity is increased stepwise according to a partially constant, increasing function.

The variants a and b require a filter for separating the probe tone and the stimulus tone, as do all other variants where the stimulus tone is continuous and a probe tone is used for probing the reaction of the stapedius to the stimulus tone.

The curve c represents a variant in which the stimulus tone is a pulse signal. However, the interval between the pulses is so brief that the degree of contraction of the stapedius essentially does not have time to change between the pulses.

In one preferred mode of implementation, the pulses have a duration of about 50 ms and the time elapsing between the pulses is about 70 ms. If there is a much longer interval between the pulses, for instance exceeding 200 ms, the stapedius has time to begin to relax. This will then create discontinuities in the otherwise continuous curve, which makes it more difficult to define the development and inclination of the curve and lessens the accuracy of the establishment of the stimulus threshold of the stapedius reflex.

The stimulus tone according to the curve c comprises a sequence of pulses of increasing intensity. For each stimulus pulse, the sound pressure level is increased in relation to that of the preceding pulse. The increase from one pulse to the next may, for instance, be about 1.5 dB. The sound pressure level of the pulses may, for example, be varied between 75 dBHL and 110 dBHL (100 dBHL for 4 kHz). The number of pulses per sequence may, for instance, be 20-22. Advantageously, the test involves one sequence of stimulus pulses for each of the frequencies 500 Hz, 1000 Hz, 2000 Hz and 4000 Hz.

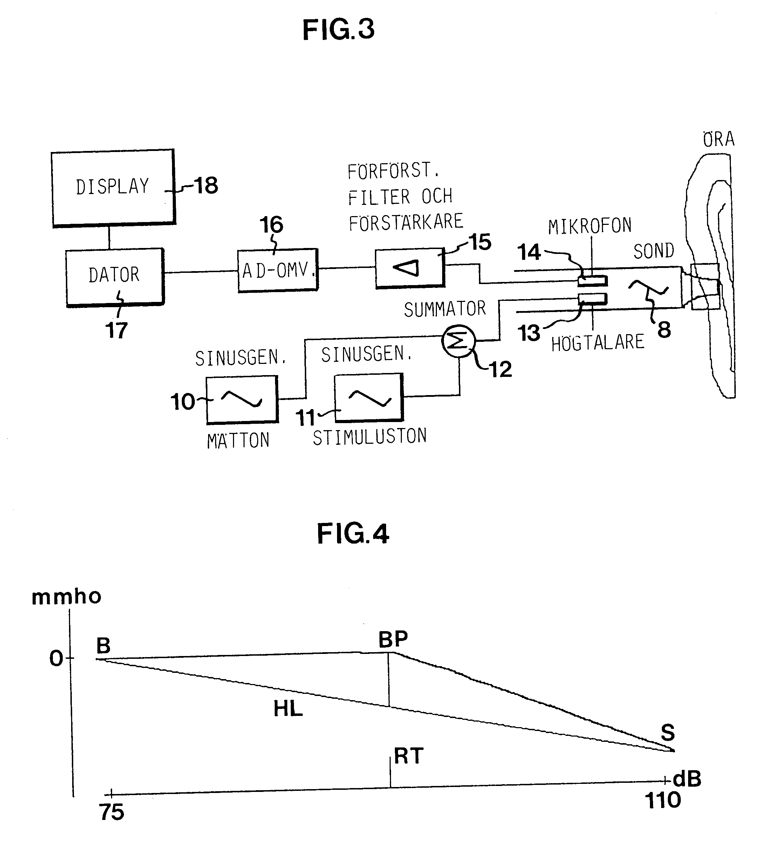

Fig. 3 is a block diagram showing one embodiment of the impedance audiometer according to the invention. A first sinusoidal generator 10 for generating a probe tone and a second sinusoidal generator 11 for generating a stimulus tone are, via an adder 12, connected to a loudspeaker 13 in a probe 8. Further, the probe is provided with a microphone 14 which, via a filtering and amplifying unit 15, is connected to an analog-to-digital converter 16, which in turn is connected to a computer 17 controlling the impedance audiometer and having a display 18 for displaying the test results.

The impedance audiometer operates as follows. The two sinusoidal generators 10 and 11 generate electric signals which are added up by the adder 12 and converted by the loudspeaker 13 to the acoustic signals constituting the stimulus tone and the probe tone. The adder 12 may be dispensed with, but use then has to be made of two loudspeakers, one for the stimulus tone and one for the probe tone. When the stimulus tone is pulsed, it may be sufficient to have one sinusoidal generator, which alternately generates the probe tone and the stimulus tone.

The microphone 14 registers the sound pressure generated by the probe tone in the auditory meatus and converts the sound to electric signals, which are then amplified and filtered. The filtered signal is A/D-converted and is processed by the computer 17, whereupon the result is displayed on the display 18. If the stimulus tone is continuous, the filtering primarily serves to separate the probe tone from the stimulus tone. If the stimulus tone is pulsed, the filtering merely serves to filter off any outside, disturbing noise, in which case much lower requirements are placed on the filter.

The computer 17 controls the whole testing procedure automatically and, for instance, activates the stimulus tone and varies its intensity and frequency.

Fig. 4 is a diagram illustrating the test results. The vertical axis represents the admittance of the middle-ear system, and the horizontal axis represents the sound pressure level of the stimulus tone. As appears from the curve in the diagram, the sound pressure level of the stimulus pulses has increased from 75 dB to about 93 dB without any reaction from the stapedius. At 93 dB, the first reaction can be perceived as a downward deflection of the curve. Once the stapedius reflex has been triggered, the stapedius will contract in a manner proportional to the sound pressure level of the stimulus tone, the eardrum being correspondingly stiffened and the admittance of the middle-ear system being reduced, which in turn increases the sound pressure in the auditory meatus.

In order that it should be possible to establish as accurate a value as possible for the stimulus threshold of the stapedius reflex, i.e. the break point BP of the curve, use is made of the following method, which is automatically implemented by the computer. An auxiliary line HL is drawn between the beginning B and the end S of the curve. Then, the vertical distance between the curve and the auxiliary line is determined at each point, the intensity value of the maximum distance corresponding to the stimulus threshold of the stapedius reflex. The computer may display either the entire diagram on the display 18 or merely a numerical value of the stimulus threshold. Alternatively, the establishment can be performed with the aid of the impedance of the middle-ear system, in which case the gradually increasing degree of contraction of the stapedius is reflected as an increase of the impedance of the middle-ear system.

Finally, it should be pointed out that there are two different ways of performing the test, the stapedius reflex being triggered bilaterally, i.e. in both ears, also when the stimulation is unilateral. In the mode of implementation described in the foregoing, the stimulation is ipsilateral, i.e. in the ear where probing is carried out. However, the stimulation may also be contralateral, i.e. in the ear not subjected to the measurement.

## Claims

1. A method for testing the reflex of the stapedius, comprising the steps of
generating a stimulus tone of variable intensity and subjecting at least one ear of the test subject to this tone, and
probing the reaction of the stapedius to the stimulus tone,
**characterised** by varying the intensity of the stimulus tone in such a manner that the degree of contraction of the stapedius is essentially maintained or increased during the test.

2. A method as claimed in claim 1, **characterised** in that the ear is subjected to a continuous stimulus tone, whose intensity is varied according to an increasing function.

3. A method as claimed in claim 2, **characterised** in that the increasing function is a strictly increasing, continuous function.

4. A method as claimed in claim 2, **characterised** in that the increasing function is a partially constant function.

5. A method as claimed in claim 1, **characterised** in that the stimulus tone is a pulse signal.

6. A method as claimed in claim 5, **characterised** in that the time elapsing between two successive pulses is less than 200 ms, preferably less than 100 ms.

7. A method as claimed in any one of the preceding claims, **characterised** in that the reaction of the stapedius to the stimulus tone is probed by generating a probe tone, to which is subjected at least one ear of the test subject, and measuring the sound pressure generated by the probe tone in the ear subjected thereto.

8. A method as claimed in claim 7, **characterised** in that the sound pressure generated by the probe tone is measured during the interval between the pulses of the stimulus tone.

9. A device for testing the reflex of the stapedius, comprising
means (11) for generating a stimulus tone of variable intensity,
means (13) for subjecting at least one ear of the test subject to the stimulus tone, and
means (14) for probing the reaction of the stapedius to the stimulus tone,
**characterised** by means (17) for controlling the variation of the intensity of the stimulus tone in such a manner that the degree of contraction of the stapedius is essentially maintained or increased during the test.

10. A device as claimed in claim 9, **characterised** in that the control means (17) are adapted to cause the intensity of the stimulus tone to vary according to an increasing function.

11. A device as claimed in claim 9, **characterised** in that the tone-generating means (11) are adapted to generate the stimulus tone in the form of a pulse signal.

12. A device as claimed in any one of the preceding claims, **characterised** in that it further comprises means (10) for generating a probe tone, as well as means (13) for subjecting at least one ear of the test subject to the probe tone, and that the means (14) for probing the reaction of the stapedius to the stimulus tone include means for measuring the sound pressure generated by the probe tone in the ear subjected thereto.

13. A device as claimed in claim 12, **characterised** in that the means (14) for measuring the sound pressure generated by the probe tone are adapted to perform this measurement during the interval between the pulses of the stimulus tone.
